(19) Europäisches Patentamt / European Patent Office / Office européen des brevets

(11) **EP 4 252 684 B1**

(12) **EUROPEAN PATENT SPECIFICATION**

(45) Date of publication and mention
of the grant of the patent:
**17.07.2024   Bulletin 2024/29**

(21) Application number: **23165733.9**

(22) Date of filing: **30.03.2023**

(51) International Patent Classification (IPC):
***A61B 17/32*** *(2006.01)*      ***A61B 18/12*** *(2006.01)*

(52) Cooperative Patent Classification (CPC):
**A61B 17/320068;** A61B 18/1206;
A61B 2017/00017; A61B 2017/00199

(54) **SURGICAL GENERATOR WITH IMPROVED DRIVING OF ULTRASONIC SURGICAL INSTRUMENT**

CHIRURGISCHER GENERATOR MIT VERBESSERTEM ANTRIEB EINES CHIRURGISCHEN ULTRASCHALLINSTRUMENTS

GÉNÉRATEUR CHIRURGICAL À COMMANDE AMÉLIORÉE D'INSTRUMENT CHIRURGICAL À ULTRASONS

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority:  **31.03.2022   US 202263326039 P**

(43) Date of publication of application:
**04.10.2023   Bulletin 2023/40**

(73) Proprietor: **Olympus Winter & Ibe GmbH
22045 Hamburg (DE)**

(72) Inventors:
• **Dijkstra, Jelle
12205 Berlin (DE)**
• **Dietrich, Stefan
14469 Potsdam (DE)**
• **Becker, Dimitri
10243 Berlin (DE)**

(74) Representative: **Glawe, Delfs, Moll
Partnerschaft mbB von
Patent- und Rechtsanwälten
Postfach 13 03 91
20103 Hamburg (DE)**

(56) References cited:
**US-A- 5 151 085**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

The invention relates to a surgical generator configured to output a high-frequency alternating voltage to a surgical ultrasound instrument. It comprises an inverter for generating high-frequency voltage, particularly in the ultrasound frequency range, which is provided at an output socket for connection of the surgical instrument.

In surgery, surgical ultrasound instruments are employed in all fields of surgery for cutting of tissue and sealing of vessels, even larger vessels. An advantage of ultrasound surgeries is that at a minimum of thermal spread is generated, thereby minimizing adverse impact on adjacent tissue. Various different ultrasound instruments are used dependent on the actual task at hand.

Surgical ultrasound instruments comprise an ultrasonic transducer converting electrical energy provided by the surgical generator into ultrasonic energy which then can be used for therapy. Generally speaking, ultrasound instruments are a complex load to the surgical generator. The electric impedance is complex and dependent on frequency, and moreover significant resonances are experienced corresponding to mechanical resonance modes. The mechanical resonance itself is affected by the mechanical load actually present at the surgical instrument, which in turn depends on the kind of tissue treated and can therefore change quite dynamically during application of the surgical instrument.

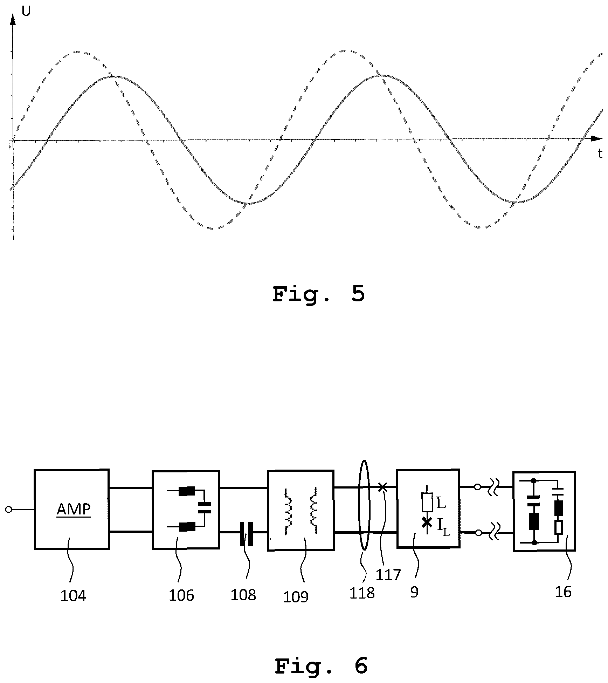

For driving of ultrasound instruments, the surgical generator requires an inverter or amplifier that has an adjustable frequency, e.g. a class-D amplifier. In order to adapt the output of the amplifier to the actual ultrasound instrument in use a matching circuit is required. The matching circuit typically consists of a parallel inductor named matching coil connected in parallel to the surgical instrument and being used to shape the impedance characteristic of the instrument such that the desired mechanical resonance mode can be excited. An exemplary embodiment of such a configuration is shown in figure 6. The matching coil generally serves two purposes. First it improves the transfer function of the surgical generator, i.e. the output voltage acting on the instrument as a function of the input signal used to drive the amplifier. Second, the matching coil improves the phase measurement that is required for a phase locked-loop-circuit (PLL) typically used for following the resonance frequency as it changes in use due to changing mechanical load to the instrument.

However, matching coils are rather bulky, specific to the instrument and are therefore rather expensive. Further, being specific to the instrument means that the matching coil must be tuned to the specific transfer characteristic of the ultrasound instrument. Therefore, only those ultrasound instruments that were already available when the surgical generator and its matching coil were developed can be connected. In other words, the surgical generator is tied to instruments that already existed at its manufacturing. This is a serious limitation restricting usage of newer, more advanced ultrasound instruments. US5151085A discloses an apparatus for generating an ultrasonic oscillation with features to match the impedance between the ultrasonic transducer and the driving circuit.

It has been proposed to avoid the task of providing the matching coil by doing away with the concept of tuning to a resonance frequency by changing to a different concept of measuring static capacitance of the surgical ultrasound instrument instead (US 10,265,117 B2). However, this requires constant repetitive measurement of the static capacitance.

Thus, it is an object of the invention to provide an improved surgical generator providing better adaptability to the ultrasound instrument actually used.

The solution according to the invention resides in the features of the independent claim. Advantageous embodiments are the subject matter of the dependent claims.

In a surgical generator configured to output a high-frequency alternating voltage to a surgical ultrasound instrument, comprising a main control unit, an oscillator generating a driving oscillation and an inverter generating high-frequency alternating voltage dependent on the driving oscillation, the high frequency alternating voltage being supplied via a filter and a matching circuit to an output socket for connection of said surgical instrument, wherein a matching circuit is provided for matching power output of the inverter to said surgical instrument, it is provided according to the invention that the matching circuit is formed by a matching-coil emulation device configured to emulate a matching coil comprising (i) a correction device acting on the driving oscillation, the correction device comprising a mixing unit and a feedback circuit whose input is connected to the output socket, wherein the feedback circuit calculates a correction signal supplied as an input to the mixing unit whose other input receives the driving oscillation, an output of the mixing unit being supplied to the inverter; and (ii) an artificial phase generator configured to provide an artificial phase signal input to the oscillator, the artificial phase generator comprising an estimator for a virtual current in consideration of the emulated matching-coil and being configured to determine a phase shift between said virtual current and the measured output voltage, the artificial phase being different from a measured phase shift between output voltage and current.

The invention aims at providing a specific matching circuit thereby replacing the physical matching coil. The invention realized that it requires a combination of two different features in order to provide such a matching circuit that is enabled to replace the physical matching coil. Firstly, the oscillation used in order to drive the inverter generating the output voltage must be modified by the correction device dependent on the state of the actual electrical parameters like voltage and current at the output socket. The mixing unit alters the signal emitted by the oscillator dependent on the output of the feedback circuit, e.g. the time varying output value of the feedback circuit is subtracted (or added) to the time varying driving oscillation, thereby providing a modified oscillation for driving of the inverter. To this end, the mixing unit is preferably configured to provide real-time mixing (e.g. by subtraction or addition) of time varying signals. A core aspect is, that by means of the feedback circuit and the mixing unit a shaping of the transfer function of the amplifier and the filter between the amplifier and the output can be achieved, thereby employing the filter to mimic the effect of the matching coil. By virtue of this, the filter which is needed anyway can be used in order to make the matching coil superfluous.

However, this could not work alone. Therefore, as a second key aspect it is necessary to generate the signal required for the correction device. With a physical matching coil, the combination of the actual load as experienced by the surgical instruments in combination with the physical matching coil would form a complex impedance leading to a certain phase difference of the current to the output voltage. However, lacking a physical matching coil, this phase difference cannot be measured as it depends also on the current through the matching coil which is not present. The invention thus provides for an artificial phase generator, which uses actual voltage as measured and a virtual current that combines actual output with an imaginary current through the (non-existent) matching coil. To this end, an estimator is provided that is configured to determine the virtual current which would flow as output current if the matching coil were present. By the combination of these elements the current can be determined that would be present if there was a properly dimensioned matching coil, and according to this virtual current is calculated.

Thereby, the missing current signal can be provided for determining a phase shift between actual measured voltage and the current in consideration of a properly dimensioned, but nonexistent matching coil; and a signal for this phase shift can be used to drive the correction circuit properly such as to achieve a transfer function emulating the (non-existing) matching coil. As a result, due to a reshaped transfer function by virtue of the correction device, the ultrasonic instrument receives the same voltage and current at the same phase shift as it would be if there was present the conventional, bulky and expensive matching coil. Due to this electronic emulating, this emulation is flexible and works also properly with newly developed surgical ultrasound instruments having different electrical characteristics, as opposed to matching coils which must always be tuned to the specific surgical ultrasound instrument and its electrical characteristic.

The invention thus achieves significant improvements with respect to different key aspects, namely further it is more versatile in terms of ultrasound instruments to be used and further it reduces bulk and cost of the surgical generator itself while improving flexibility in terms of ultrasound instruments that can be used, even newer ones.

In the context of the present application the term "high-frequency" relates to frequencies in the ultrasound frequency range generated by the surgical generator, typically in the range of 20 kHz to 200 kHz (ultrasound surgical generators).

The inverter is a device to provide the actual high-frequency output for the surgical ultrasound instrument to be connected to the output socket. The term inverter is rather broad and comprises actual inverter technology as well as converters and amplifiers.

The filter is located between an output of the inverter and the output socket, thereby separating the load from the output of the inverter. Typically, the filter comprises aqn in-line inductance, and further may comprise a parallel capacitor (LC-filter).

The matching coil emulation device is configured to emulate a matching coil which would conventionally be present in order to match the instrument to the surgical generator. Being an emulation device, the matching coil emulation device itself does not comprise a matching coil; it thus may be said that it is coil-less.

In the context of the present invention, an estimator is understood to be a device designed to estimate the state of a system from measurements of its outputs. It is configured to estimate a variable or state that is not, or at least not directly, accessible by using parameter and other measured variable outputted by the system. Various such estimators are known to the person skilled in the art of control systems. Non-limiting examples are observers in state-space or Wiener-Filter and Kalman-Filter.

In a preferred embodiment, the feedback circuit is configured as a state feedback. Thereby, the benefits achieved in control engineering by implementing state space can be used in an advantageous manner.

Preferably, the feedback circuit may be configured to be supplied with a signal representative for a measured output voltage and/or current as an input to the feedback circuit. Measuring output voltage and/or current is achievable in a reliable manner with a minimum of effort, in particular in consideration that for the sake of power regulation by the main control unit typically sensors for voltage and current at the output are provided anyway. So no additional cost in terms of sensors is involved by using for the feedback circuit a measured output voltage (or current) signal that is measured anyway.

Advantageously, the feedback circuit is configured as a negative feedback comprising a preselectable amplification factor which may be applied at an input of the feedback circuit. Such an amplification factor, which may also be termed k-factor, can be utilized to implement an uncomplicated and efficient feedback circuit. Preferably, the amplification factor is determined according to a ratio of filter inductivity to an inductivity value of a matching coil matched to the surgical instrument. That inductivity value is the inductivity of a physical matching coil (which is to be emulated by the present invention) would have in order to be matched to the surgical instrument. Further preferably, this ratio is further modified by the ratio of the current flowing through an in-line inductivity of the filter to the current expected to be flowing through the physical matching coil (if it were present). By employing such a ratio, also any effect of a transformer in the output line is considered, too.

Advantageously, the feedback circuit, in particular an amplification factor thereof, is set automatically according to a type of the surgical instrument. Thereby the user is freed from the task of determining the amplification factor. However, this does not rule out a manual interaction for setting the amplification factor, e.g. by the user selecting the type of instrument manually and the amplification factor thereby being set, e.g. by means of a look-up table.

Preferably, the feedback circuit is configured to output a wave signal having the same frequency as the driving oscillation but different amplitude and/or phase then said driving oscillation. By such a configuration the wave signal can be utilized rather directly for the correction device, without any need of preprocessing. This further simplifies the correction device and its operation.

In a preferred embodiment, the oscillator comprises a frequency following circuit configured to follow a frequency of the voltage at the output socket. By virtue of such a following the oscillator tracks any variation of the resonance frequency experienced at the output socket due to changing loads, in particular changing mechanical loads of the surgical ultrasound instrument connected at the output socket. Preferably, the frequency following unit comprises a phased-locked-loop circuit (PLL). This allows for an efficient and reliable tracking of the frequency at the output socket. Thereby safe and stable operation conditions can be achieved.

Further preferably, the frequency following unit is controlled by the phase signal generated by the artificial phase generator. This enables the frequency following unit to track the frequency and phase shifts at the output socket even if a direct measurement of the phase is not possible. This also addresses the situation in which an actual measurement is not meaningful, i.e. an actually measured phase does not represent the phase information required for proper operation of the correction device. This is particularly useful if the phase information concerning the phase shift is artificially generated, e.g. by employing an estimator for the amplitude and phase of a current which is not directly measurable, like a virtual current.

Advantageously, the estimator for the virtual current comprises a parameter representative of the inductivity of the matching coil to be emulated. By employing such parameter, a more precise estimation can be achieved of the virtual current that would flow as output current if the matching coil were present and be connected in parallel to the surgical instrument. This parameter is preferably set depending on the type of the surgical ultrasound instrument connected to the output socket.

This can be made manually or automatically. Preferably, the main control unit is configured to set this parameter. This comprises the main control unit being configured to determine the type of surgical instrument by means of a user input device, and/or to determine the type of the surgical instrument automatically by identifying the surgical ultrasound instrument, e.g. by reading out instrument data of the surgical ultrasound instrument. Thereby an efficient and easy to use way for setting the inductivity parameter is realized, which in case of automatic determination by the main control unit eliminates any risk of user error. This further enhances reliability and ultimately improves safety for the patient.

The invention is explained in more detail below with reference to an advantageous exemplary embodiment. In the figures:

Fig. 1      shows a surgical generator according to an exemplary embodiment with an attached electrosurgical instrument;

Fig. 2      shows a block diagram of the surgical generator shown in figure 1;

Fig. 3a, b      transfer function showing gain and phase of the system for no matching coil, physical matching coil and emulated matching coil according to the invention;

Fig. 4a, b      instrument output impedance for no matching coil, physical matching coil and emulated matching coil according to the invention;

Fig. 5      driving oscillation without and after modifying by a correction device; and

Fig. 6      shows a block diagram of a power section from an inverter to an ultrasound instrument connected at an output socket according to the prior art.

The invention will be explained with respect to an exemplary embodiment of a surgical generator according to the invention. This exemplary embodiment is a surgical ultrasound generator that is identified as a whole by reference sign 1. The surgical ultrasound generator 1 comprises a housing 11 provided with a general-purpose user interface 14, a power selector knob 12 and an instrument type selector knob 13 as well as an output socket 15 for an surgical ultrasound instrument 16. The surgical ultrasound instrument 16 is connected via a cable to the output socket 15 of the surgical ultrasound generator 1.

The ultrasound surgical generator 1 is supplied by a mains cable 21 which can be connected by means of plug 20 at its end to a public electricity grid (not shown) or other suitable means of electrical supply. Thereby, electrical power is supplied to the surgical ultrasound generator 1.

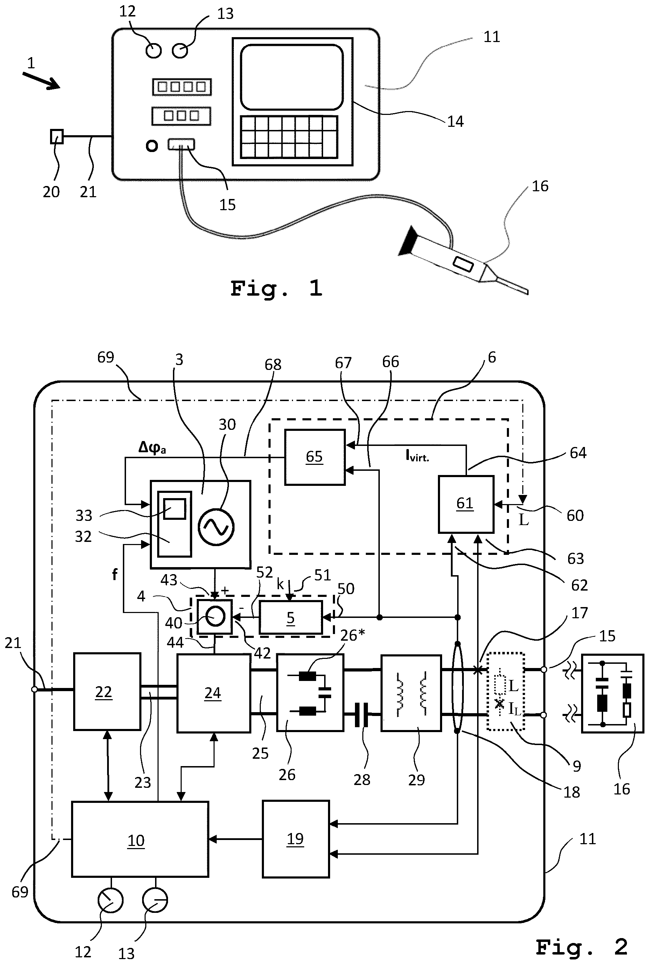

A schematic functional diagram of the electrosurgical generator 1 is illustrated in figure 2. It comprises in a housing 11 a power supply unit 22 that is supplied with electrical power by the mains connecting cable 21 (see figure 1). The power supply unit 22 comprises a rectifier and feeds a DC link 23 with direct voltage, the DC link 23 feeding an inverter 24. The inverter 24 is in the exemplified embodiment an amplifier, preferably of a class-D type. Based on a high-frequency driving oscillation 43 supplied by a driving oscillator 3 the inverter 24 amplifies the supplied high-frequency oscillation and generates an according high-frequency alternating voltage of about 10 to 100 Volt, typically about 50 Volt. The high-frequency is variable and in the ultrasound range which ranges between 20 and 60 kHz, typically in the range between 40 and 55 kHz.

The high-frequency voltage generated by the inverter 24 is fed to an output line 25. Subsequent to the inverter 24 a filter 26 is provided which is a filter of the LC type having an in-line inductivity 26* in either line of the output line 25 and a capacitor connecting both in-line inductivities. Subsequent to the filter 26 and an optional DC blocking capacitor 28 an isolation transformer 29 is arranged which provides for isolation and stepping up of the output voltage, the output transformer having a transmission ratio of e.g. 11. The resulting output voltage and current are sensed by a current measuring device 17 and a voltage measuring device 18 and are routed to the output socket 15 into which the surgical ultrasound instrument 16 can be plugged. The configuration from the inverter 24 to the output socket 15 forms the power section of the surgical ultrasound generator 1.

Operation of the surgical ultrasound generator 1 is controlled by a main control unit 10. It is connected to the power supply unit 22 and the inverter 24 by means of signaling lines. Further, a power feedback circuit 19 is provided to which the current and voltage measuring devices 17, 18 are connected as inputs. The feedback device 19 determines output power based on these inputs and outputs a signal representing actual outputted power to the main control unit 10, which in turn provides control signals to the inverter 24 in accordance with functions and modes set by the user by a user interface 14 and/or power selection knob 12. It further controls initial frequency f of the driving oscillator 3. This configuration described hitherto is generally known in the art and will not be further explained for the sake of brevity.

Surgical ultrasound instruments 16 comprise an ultrasound transducer (not shown) that converts electrical energy into ultrasound energy. The electric impedance of the ultrasound instrument 16 corresponds to mechanical resonance modes of the surgical ultrasound instrument 16. This mechanical resonance is affected by the mechanical load to the ultrasound instrument which is variable, e.g. depends on the tissue to be worked on by the ultrasound instrument.

As a reference, in figure 6 a power section according to the prior art beginning at the amplifier acting as inverter 104 is shown in figure 6. Subsequently arranged are the LC filter 106, DC blocking capacitor 108, the isolation transformer 109, current and voltage measuring devices 117 and 118 as well as a matching coil 9 and finally the output socket 115 into which the surgical ultrasound instrument 16 is to be plugged. In order to drive the ultrasound instrument 16 properly, a matching circuit is required which consists of a parallel inductor termed "matching coil" and identified in figure 6 by reference numeral 9. Said matching coil is used to shape the impedance characteristic of the instrument 16 such that the desired mechanical resonance mode can be excited. The matching coil 9 which ordinarily is a part of the ultrasound surgical generator must thus be matched to the actual instrument 16. If another instrument 16 shall be connected to the ultrasound surgical generator, a second matching coil is required or the matching coil 9 must be a switchable matching coil enabled to be reconfigured such as to match said another instrument.

The invention aims at abolishing the matching coil 9. Consequently, the matching coil 9 is not existent in the exemplary embodiment as shown in the figures, and in particular in figure 2 the abolished matching coil 9 is shown in phantom lines. According to the invention, the abolished matching coil 9 is replaced by a matching coil emulation device that is configured to emulate the abolished matching coil 9. To this end, the matching coil emulation device comprises a correction device 4 configured to perform corrections on the driving oscillation formed by the oscillator 3 and an artificial phase generator 6 comprising an estimator 61 for a virtual current in consideration of the matching coil that is emulated.

The estimator 61 is configured to determine said virtual current as an output current, and based thereon the artificial phase generator 6 calculates a phase shift between said virtual current and the measured output voltage. To this end, the actual voltage as measured by voltage sensor 18 is supplied to a first input 62 of the estimator 61. Further, the actual current measured by current sensors 17 is supplied to a second input 63 of the estimator 61. Further, as an additional input a parameter 60 representing the inductivity value "L" of the non-existent matching coil is set. Based on this, the estimator 61 is configured to determine the (complex) virtual current $I_{virt.}$ according to

$$I_{virt.} = I_{meas.} + \frac{U_{meas.}}{j\omega L}$$

wherein $I_{meas.}$ is the current measured by the current sensors 17 and $U_{meas.}$ is the voltage measured by voltage sensor 18. The value of the virtual current $I_{virt.}$ is provided at output 64 and supplied to an input 67 of a phase shift calculator 65, and to another input 66 a measured voltage signal as measured by the voltage sensor 18 is supplied. Based on these inputs there, the phase shift calculator 65 determines a phase shift between voltage and current, namely measured voltage $U_{meas.}$ and the virtual current $I_{virt.}$. A signal for this phase shift $\Delta\varphi_a$ is output at line 68 from the artificial phase generator 6. This signal for the artificial phase difference $\Delta\varphi_a$ is supplied to an input of a PLL 33 for frequency following of the oscillator 3.

It is worth to note that this phase shift calculated by the artificial phase generator 6 - due to considering additionally the imaginary current through the emulated matching coil - is and must be different from the actual phase shift defined by actual voltage current and voltage as measured by current and voltage sensors 17, 18.

The oscillator 3 comprises a base generation unit 30 which is generating a sinewave oscillation in the depicted embodiment. The frequency of the generated oscillation is determined by the frequency following unit 32 to which the control signal for an initial frequency f is supplied by the main control unit 10. Based on this initial frequency the oscillator 3 emits the driving oscillation at its output 34 which is supplied via a correction device 4 to the inverter 24 which acts as an amplifier. The frequency following unit 32 further comprises said phased-locked-loop (PLL) circuit 33. It receives the phase difference signal difference $\Delta\varphi_a$ via the line 68 from the artificial phase generator 6. By virtue of this, the PLL circuit 33 keeps the oscillator 3 at a constant phase shift between the measured output voltage and current, and further the oscillator 3 tracks any changes in the resonance frequency as they are experienced in the course of using the surgical ultrasound instrument 16 due to varying load conditions.

Further, the transfer function of the power section beginning with the inverter 24 is amplifier is shaped by using the correction device 4. To this end, a feedback circuit 5 is provided having an input 50 to which a voltage signal $V_{meas.}$ as measured by the voltage sensors 18 is provided. Further, as an additional input 51 a parameter "k" is provided at the feedback circuit 5.

The "k" parameter can be determined by the reciprocal of an inductivity L the matching coil 9 would have if it were present times the in-line inductance 26* which is to be doubled in this case since in both lines such an in-line inductance 26* exists. Further, in consideration of the isolation transformer 29 a ratio between the current in the in-line filter inductance 26* and the current flowing through the matching coil 9 (if it were present) can be determined and in the present case said ratio is N = 11. Assuming that the inductivity L of the matching coil 9 would be 3.3 mH and the in-line inductance 26* has 6.25 μH each, the "k" parameter can be determined to be:

$$k = \frac{2 \cdot 6.25\,\mu H}{3.3\,mH} \cdot 11 \approx 0{,}042$$

Based on this, the feedback circuit 5 determines a correction value according to

$$-k \cdot V_{meas.}$$

at an output 52 of the feedback circuit 5. This is supplied to a second input 42 of a mixing unit 40 of the correction device 4. At the first input 43 of the mixing unit 40 the driving oscillation is supplied as output by the oscillator 3. Accordingly, the driving oscillation as outputted by the oscillator 3 is corrected by the output of the feedback circuit 5, and the resulting signal is supplied via line 44 to an input of the inverter 24 for amplification and outputting to output line 25.

Thus, as a result the transfer function is shaped by the correction device 4 and the phase shift between voltage and current at the output socket 15 is modified by the artificial phase generator 6. The resulting corrected oscillation supplied by line 44 is different in amplitude and phase from the original driving oscillation as outputted by the oscillator 3. This is shown in figure 5, where a dashed line represents the original driving oscillation and a solid line represents the corrected oscillation which is shaped according to the present invention and is supplied by line 44 to the inverter 24 for amplification.

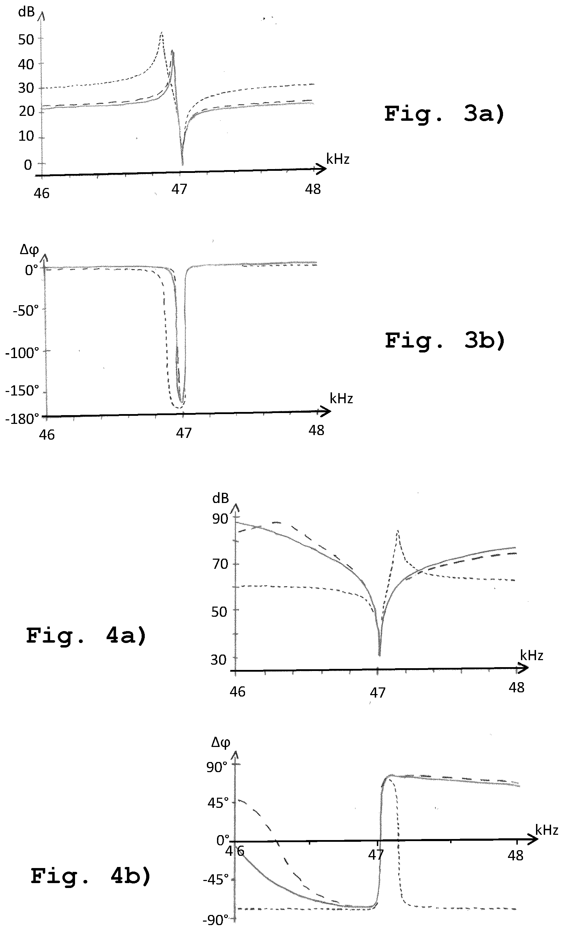

The effect of the correction device 4 and the artificial phase generator 6 is illustrated in figures 3 and 4. Figure 3a shows gain and figure 3b shows phase shift depending on frequency f at the output socket 15. The dashed line shows the transfer function of a power section according to the prior art with the physical matching coil 9 being present. By a mere deletion of the matching coil a transfer function will result as it is shown by the dotted line which is considerably different in terms of gain and even more in terms of phase. By providing a matching coil emulation device comprising the correction device 4 and the artificial phase generator 6 as described before, a transfer function will result as shown by the solid line. It can be readily appreciated, that the solid line closely tracks and to a large extent overlaps the dashed line of the physical matching coil.

Similarly, in figure 4a and 4b magnitude and phase of the output impedance of the surgical ultrasound instrument 16 are shown. The output impedance with the physical matching coil 9 present according to the prior art is shown by a dashed line. A scenario in which the matching coil 9 is just removed is depicted by the dotted line. It can be readily appreciated that this line is much different and in particular provides a significant deviation in phase, leading to a second root at a frequency above the resonance frequency of 47 kHz, which is detrimental to stability. All this is cured by providing the matching coil emulation according to the present invention leading to an output impedance as shown by the solid line which is much closer to the dashed line in particular in vicinity of the critical resonance frequency around 47 kHz. Therefore, it can be readily appreciated that the matching coil emulation device produces excellent results in replacing the physical matching coil 9

Accordingly, by virtue of the combination of the correction device 4 with the artificial phase generator 6 the desired effect is achieved of emulating the (physically non-existent) matching coil 9 in order to properly drive the surgical ultrasound instrument 16.

Further, in order to be capable of driving properly other types of surgical ultrasound instruments 16 requiring a different matching coil 9 to be emulated, the parameter "L" can be preselected to a different value by means of the main control unit 10 by a line 69. The type of the surgical ultrasound instrument 16 used can be selected by the user by means of knob 13 attached to the main control unit 10. Optionally, the main control unit may be set up to read out the type directly from the surgical ultrasound instrument 16 by means of a communication interface (not shown) at the output socket 15. Either way, it is therefore achieved to readily adapt the ultrasound surgical generator 1 to different types of surgical ultrasound instruments 16, even such instruments being released after the ultrasound surgical generator has been manufactured. This greatly improves versatility.

**Claims**

1. Surgical generator configured to output a high-frequency alternating voltage to a surgical ultrasound instrument (16), comprising a main control unit (10), an oscillator (3) generating a driving oscillation and an inverter (24) generating high-frequency alternating voltage dependent on the driving oscillation, the high frequency alternating voltage being supplied via a filter and a matching circuit to an output socket (15) for connection of said surgical instrument (16), wherein a matching circuit is provided for matching power output of the inverter (24) to said surgical instrument (16),

   **characterized in that**,

   the matching circuit is formed by a matching-coil emulation device configured to emulate a matching coil comprising a correction device (4) acting on the driving oscillation, the correction device (4) comprising a mixing unit (40) and a feedback circuit (5) whose input (50) is connected to the output socket (15), wherein the feedback circuit (5) calculates a correction signal supplied as an input (42) to the mixing unit (4) whose other input (43) receives the driving oscillation, an output (44) of the mixing unit (40) being supplied to the inverter (24); and an artificial phase generator (6) configured to provide an artificial phase signal as an input to the oscillator (3), the artificial phase generator comprising an estimator (61) for a virtual current in consideration of the emulated matching-coil and being configured to determine a phase shift between said virtual current and the measured output voltage, the artificial phase being different from a measured phase shift between output voltage and current.

2. The surgical generator of claim 1, wherein the feedback circuit (5) is configured as a state feedback.

3. The surgical generator of claim 1 or 2, wherein a signal representative for measured output voltage and/or output current is supplied at an input (50) to the feedback circuit (5).

4. The surgical ultrasound generator of any of the preceding claim, wherein the feedback circuit (5) is configured as a negative feedback and comprises a preselectable amplification factor.

5. The surgical ultrasound generator of the preceding claim, wherein the amplification factor is determined according to a ratio of filter inductivity (26*) to inductivity of a matching coil (9) matched to the surgical instrument (16) .

6. The surgical ultrasound generator of any of the preceding claims, wherein the feedback circuit (5), in particular an amplification factor thereof, is set automatically according to a type of the surgical instrument (16).

7. The surgical ultrasound generator of any of the preceding claims, wherein the feedback circuit (5) is configured to output a wave signal having the same frequency as the driving oscillation but different amplitude and/or phase than said driving oscillation.

8. The surgical generator of any of the preceding claims, wherein the oscillator (3) comprises a frequency following unit(32) configured to follow a frequency of the voltage at the output socket (15).

9. The surgical generator of the preceding claim, wherein the frequency following unit (32) comprises a phased-locked-loop circuit (33).

10. The surgical generator of claims 8 or 9, wherein the frequency following unit (32) is controlled by the phase signal (68) generated by the artificial phase generator (6) .

11. The surgical generator of any of the preceding claims, wherein the estimator (61) for the virtual current comprises a parameter representative of an inductivity, L, of the matching coil (9) to be emulated.

12. The surgical generator of the preceding claim, wherein said parameter representative for the inductivity, L, is pre-selectable.

**13.** The surgical generator of the preceding claim, wherein the main control unit (10) is configured to set the parameter representative for the inductivity depending on a type of the surgical instrument (16) connected to the output socket (15).

**14.** The surgical generator of any of the preceding claims, wherein the main control unit (10) is configured to determine the type of the surgical instrument (16) by means of a user input device (13) and/or automatically by reading out instrument data of the surgical instrument (16).

**Patentansprüche**

**1.** Chirurgischer Generator, der ausgestaltet ist, um eine hochfrequente Wechselspannung an ein chirurgisches Ultraschallinstrument (16) auszugeben, umfassend eine Hauptsteuereinheit (10), einen Oszillator (3), der eine Antriebsoszillation generiert, und einen Wechselrichter (24), der hochfrequente Wechselspannung in Abhängigkeit von der Antriebsoszillation generiert, wobei die hochfrequente Wechselspannung über ein Filter und eine Anpassungsschaltung einer Ausgangsbuchse (15) zur Verbindung mit dem chirurgischen Instrument (16) zugeführt wird, wobei eine Anpassungsschaltung zum Anpassen der Leistungsausgabe des Wechselrichters (24) an das chirurgische Instrument (16) bereitgestellt wird, **dadurch gekennzeichnet, dass**

die Anpassungsschaltung durch eine Anpassungsspulenemulationsvorrichtung gebildet ist, die ausgestaltet ist, um eine Anpassungsspule zu emulieren, umfassend eine Korrekturvorrichtung (4), die auf die Antriebsoszillation einwirkt, wobei die Korrekturvorrichtung (4) eine Mischeinheit (40) und eine Feedbackschaltung (5) umfasst, deren Eingang (50) mit der Ausgangsbuchse (15) verbunden ist, wobei die Feedbackschaltung (5) ein Korrektursignal berechnet, das der Mischeinheit (4) als Eingang (42) zugeführt wird, der andere Eingang (43) der Mischeinheit die Antriebsoszillation empfängt, wobei ein Ausgang (44) der Mischeinheit (40) dem Wechselrichter (24) zugeführt wird; und einen künstlichen Phasengenerator (6), der ausgestaltet ist, um ein künstliches Phasensignal als Eingang an den Oszillator (3) bereitzustellen, wobei der künstliche Phasengenerator einen Schätzer (61) für einen virtuellen Strom unter Berücksichtigung der emulierten Anpassungsspule umfasst und ausgestaltet ist, um eine Phasenverschiebung zwischen dem virtuellen Strom und der gemessenen Ausgangsspannung zu bestimmen, wobei die künstliche Phase sich von einer gemessenen Phasenverschiebung zwischen Ausgangsspannung und -strom unterscheidet.

**2.** Chirurgischer Generator nach Anspruch 1, wobei die Feedbackschaltung (5) als Zustandsfeedback ausgestaltet ist.

**3.** Chirurgischer Generator nach Anspruch 1 oder 2, wobei ein Signal, das für gemessene Ausgangsspannung und/oder gemessenen Ausgangsstrom repräsentativ ist, der Feedbackschaltung (5) an einem Eingang (50) zugeführt wird.

**4.** Chirurgischer Ultraschallgenerator nach einem der vorhergehenden Ansprüche, wobei die Feedbackschaltung (5) als Negativfeedback ausgestaltet ist und einen vorwählbaren Verstärkungsfaktor umfasst.

**5.** Chirurgischer Ultraschallgenerator nach dem vorangehenden Anspruch, wobei der Verstärkungsfaktor gemäß einem Verhältnis von Filterinduktivität (26*) zu Induktivität einer Anpassungsspule (9) bestimmt wird, die an das chirurgische Instrument (16) angepasst ist.

**6.** Chirurgischer Ultraschallgenerator nach einem der vorhergehenden Ansprüche, wobei die Feedbackschaltung (5), insbesondere ein Verstärkungsfaktor derselben, automatisch gemäß einem Typ des chirurgischen Instruments (16) festgelegt wird.

**7.** Chirurgischer Ultraschallgenerator nach einem der vorhergehenden Ansprüche, wobei die Feedbackschaltung (5) ausgestaltet ist, um ein Wellensignal mit der gleichen Frequenz wie die Antriebsoszillation, jedoch mit einer anderen Amplitude und/oder Phase als die Antriebsoszillation auszugeben.

**8.** Chirurgischer Generator nach einem der vorhergehenden Ansprüche, wobei der Oszillator (3) eine Frequenzfolgeeinheit (32) umfasst, die ausgestaltet ist, um einer Frequenz der Spannung an der Ausgangsbuchse (15) zu folgen.

**9.** Chirurgischer Generator nach dem vorangehenden Anspruch, wobei die Frequenzfolgeeinheit (32) eine Phasenregelkreisschaltung (33) umfasst.

**10.** Chirurgischer Generator nach Anspruch 8 oder 9, wobei die Frequenzfolgeeinheit (32) durch das Phasensignal (68) gesteuert wird, das durch den künstlichen Phasengenerator (6) generiert wird.

**11.** Chirurgischer Generator nach einem der vorhergehenden Ansprüche, wobei der Schätzer (61) für den virtuellen Strom einen Parameter umfasst, der für eine Induktivität, L, der Anpassungsspule (9) repräsentativ ist, die emuliert werden soll.

**12.** Chirurgischer Generator nach dem vorangehenden Anspruch, wobei der Parameter, der für die Induktivität, L, repräsentativ ist, vorwählbar ist.

**13.** Chirurgischer Generator nach dem vorangehenden Anspruch, wobei die Hauptsteuereinheit (10) ausgestaltet ist, um den für die Induktivität repräsentativen Parameter in Abhängigkeit von einem Typ des chirurgischen Instruments (16) festzulegen, das mit der Ausgangsbuchse (15) verbunden ist.

**14.** Chirurgischer Generator nach einem der vorhergehenden Ansprüche, wobei die Hauptsteuereinheit (10) ausgestaltet ist, um den Typ des chirurgischen Instruments (16) mittels einer Benutzereingabevorrichtung (13) und/oder automatisch durch Auslesen von Instrumentdaten des chirurgischen Instruments (16) zu bestimmen.

**Revendications**

**1.** Générateur chirurgical configuré pour délivrer une tension alternative à haute fréquence à un instrument chirurgical à ultrasons (16), comprenant une unité de commande principale (10), un oscillateur (3) générant une oscillation d'entraînement et un onduleur (24) générant une tension alternative à haute fréquence dépendant de l'oscillation d'entraînement, la tension alternative à haute fréquence étant fournie par l'intermédiaire d'un filtre et d'un circuit d'adaptation à une prise de sortie (15) pour la connexion dudit instrument chirurgical (16), un circuit d'adaptation étant prévu pour adapter la puissance de sortie de l'onduleur (24) audit instrument chirurgical (16),
**caractérisé en ce que**,
le circuit d'adaptation est formé par un dispositif d'émulation de bobine d'adaptation configuré pour émuler une bobine d'adaptation comprenant :

un dispositif de correction (4) agissant sur l'oscillation d'entraînement, le dispositif de correction (4) comprenant une unité de mélange (40) et un circuit de rétroaction (5) dont l'entrée (50) est connectée à la prise de sortie (15), le circuit de rétroaction (5) calculant un signal de correction fourni en tant qu'entrée (42) à l'unité de mélange (4) dont l'autre entrée (43) reçoit l'oscillation d'entraînement, une sortie (44) de l'unité de mélange (40) étant fournie à l'onduleur (24) ; et un générateur de phase artificielle (6) configuré pour fournir un signal de phase artificielle en tant qu'entrée de l'oscillateur (3), le générateur de phase artificielle comprenant un estimateur (61) pour un courant virtuel en considération de la bobine d'adaptation émulée et étant configuré pour déterminer un déphasage entre ledit courant virtuel et la tension de sortie mesurée, la phase artificielle étant différente d'un déphasage mesuré entre la tension et le courant de sortie.

**2.** Générateur chirurgical selon la revendication 1, le circuit de rétroaction (5) étant configuré comme une rétroaction d'état.

**3.** Générateur chirurgical selon la revendication 1 ou 2, un signal représentatif de la tension de sortie mesurée et/ou du courant de sortie étant fourni à une entrée (50) du circuit de rétroaction (5).

**4.** Générateur d'ultrasons chirurgicaux selon l'une quelconque des revendications précédentes, le circuit de rétroaction (5) étant configuré comme une rétroaction négative et comprenant un facteur d'amplification présélectionnable.

**5.** Générateur d'ultrasons chirurgicaux selon la revendication précédente, le facteur d'amplification étant déterminé en fonction d'un rapport entre l'inductivité du filtre (26*) et l'inductivité d'une bobine d'adaptation (9) adaptée à l'instrument chirurgical (16).

**6.** Générateur d'ultrasons chirurgicaux selon l'une quelconque des revendications précédentes, le circuit de rétroaction (5), en particulier son facteur d'amplification, étant réglé automatiquement en fonction du type d'instrument chirurgical (16).

**7.** Générateur d'ultrasons chirurgicaux selon l'une quelconque des revendications précédentes, le circuit de rétroaction (5) étant configuré pour émettre un signal d'onde ayant la même fréquence que l'oscillation d'entraînement mais une amplitude et/ou une phase différente de ladite oscillation d'entraînement.

**8.** Générateur chirurgical selon l'une quelconque des revendications précédentes, l'oscillateur (3) comprenant une unité de suivi de fréquence (32) configurée pour suivre une fréquence de la tension au niveau de la prise de sortie (15).

**9.** Générateur chirurgical selon la revendication précédente, l'unité de suivi de fréquence (32) comprenant un circuit à verrouillage de phase (33).

**10.** Générateur chirurgical selon les revendications 8 ou 9, l'unité de suivi de fréquence (32) étant commandée par le signal de phase (68) généré par le générateur de phase artificielle (6).

**11.** Générateur chirurgical selon l'une quelconque des revendications précédentes, l'estimateur (61) du courant virtuel comprenant un paramètre représentatif d'une inductivité, L, de la bobine d'adaptation (9) à émuler.

**12.** Générateur chirurgical selon la revendication précédente, ledit paramètre représentatif de l'inductivité, L, étant présélectionnable.

**13.** Générateur chirurgical selon la revendication précédente, l'unité de commande principale (10) étant configurée pour régler le paramètre représentatif de l'inductivité en fonction d'un type d'instrument chirurgical (16) connecté à la prise de sortie (15).

**14.** Générateur chirurgical selon l'une quelconque des revendications précédentes, l'unité de commande principale (10) étant configurée pour déterminer le type de l'instrument chirurgical (16) au moyen d'un dispositif d'entrée utilisateur (13) et/ou automatiquement en lisant des données d'instrument de l'instrument chirurgical (16).

Fig. 1

Fig. 2

Fig. 3a)

Fig. 3b)

Fig. 4a)

Fig. 4b)

Fig. 5

Fig. 6

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5151085 A

- US 10265117 B2